# EUROPEAN PATENT SPECIFICATION

(11) **EP 3 024 549 B1**
(45) Date of publication and mention of the grant of the patent: **25.04.2018**
(21) Application number: 14742243.0
(22) Date of filing: 24.07.2014
(51) Int. Cl.: A61N 5/10, A61M 37/00, A61B 6/04, A61B 90/00, A61B 17/00

(54) **MEDICAL DEVICE FOR RADIOTHERAPY TREATMENT**
MEDIZINISCHE VORRICHTUNG FÜR DIE STRAHLENTHERAPIE
DISPOSITIF MÉDICAL POUR TRAITEMENT PAR RADIOTHÉRAPIE

(30) Priority: 25.07.2013 EP 13178073
(43) Date of publication of application: 01.06.2016
(73) Proprietor: Escarguel, Bruno, 83110 Sanary (FR)
(72) Inventor: Escarguel, Bruno, 83110 Sanary (FR)
(74) Representative: Novagraaf Technologies
(86) International application number: PCT/EP2014/065951
(87) International publication number: WO 2015/011239

(56) References cited:
- US-A1- 2007 167 980
- US-A1- 2008 262 518
- US-A1- 2009 125 119

## Description

### Technical field

The present invention refers to a medical device for an intracorporeal location. Therefore, the present invention has utility in the medical and surgical field.

In the description below, the references into brackets (**[ ]**) refer to the listing of references situated at the end of the text.

### Background of the Invention

State of the art cancer radiation therapy is increasingly based on the pin point application of high energy radiation which is highly tailored to the shape and position of the cancerous tumor. Modern techniques such as IMRT (intensity-modulated radiation therapy) use a pencil sized beam whose cross-section is shaped to match the tumor. This allows the physician to spare the surrounding healthy tissue while increasing the treatment dose to the cancerous target. As the size of the treatment beam decreases, the accurate location of the beam becomes much more critical. If a highly tailored beam is off target by a few millimeters, it may miss the tumor entirely.

Because of these new techniques, it becomes increasingly desirable to know the position and shape of the tumor accurately with the patient in the exact position that he will be at the time of treatment. In addition, it is critical to be able to place the patient in the same position for multiple fractions of treatment and to be able to confirm that accurate positioning has been accomplished. For this reason, manufacturers of radiation therapy machines are increasingly combining their machines with built in diagnostic imaging capability.

Advances such as On Board Imaging (OBI) and Cone Beam CT allow the verification of patient positioning in real time and the ability to confirm through x-ray that the patient is in the same position as during simulation.

It is also now common for medical clinicians to obtain high contrast and high spatial resolution CT (Computed tomography) and magnetic resonance (MR) data sets. These data sets can be obtained with high spatial resolution between contiguous image slices. These data sets allow for the reconstruction of a precise 3-dimensional (3D) model that accurately describes both the patient's external and internal anatomy. The patient specific models can be manipulated to provide reconstructed views along orthogonal or oblique planes through the patient's anatomy. These computed views allow for clinicians to carry out virtual treatments (or virtual planning) to better optimize therapy for a patient.

Virtual planning is used in several different types of therapy. Radiosurgery, stereotactic radiation therapy, and routine radiotherapy are all therapies that rely upon virtual planning to position radiation beams. Image guided surgery relies upon virtual planning to allow the surgeon to design and evaluate various surgical approaches and to target specific tissues. The virtual planning process places a unique requirement on the basic 3D image data set, that being the ability to track the patient, at the time of therapy, relative to the therapeutic tool. For radiosurgery, stereotactic radiation therapy and radiation therapy, the therapeutic tool is the radiation-generating device, most commonly, a medical linear accelerator. In the case of image guided surgery, the therapeutic tool can be one of a number of devices that the surgeon may use. For example, scalpels, biopsy needles, and operating microscopes are a few of the most commonly guided surgical tools.

In order to provide the required patient-tool tracking both the tool's position and the patient's position must be known. The most common method of tracking the patient is to place identifiable reference markers, called fiducial markers, fixed relative to the portion of the patient where treatment is desired. These markers are incorporated into the 3D image data set. Such markers are for example described in document US 2009 125 119. They are also available for identification, again on the surface of the patient (fixed relative to the portion of the patient), at the time of the therapeutic procedure. The markers on the patient are registered against their images in the 3D data set. This registration allows the computation of a rigid relationship between the virtual 3D patient and the real patient.

Once this registration has been carried out, any movement of the patient can be tracked.

This ability to potentially employ positional comparison through imaging on the treatment machine provides the opportunity to develop technologies to discretely locate the patient immobilization devices on the treatment machine and to compare the position to that of the simulation. The imaging technology in treatment and simulation do not have to be the same, and multiple imaging technologies may be employed at each stage, be it x-ray based, MRI (Magnetic resonance imaging) or other modalities. New localization techniques such as the radiofrequency technology developed by Calypso Medical Systems of Seattle present new opportunities to identify and confirm the accuracy of repeated patient positioning. Corrections may be made to the position and orientation of the patient support devices so that accurate targeting of the tumor can be achieved. In addition, the ability to align the couchtop and devices through imaging techniques on the treatment machine allow the process to be proceduralized and automated so that less time is required, increasing productivity.

Traditionally, patient treatment plans have been performed on a separate simulation machine which uses diagnostic imaging either through static images, CT (Computed tomography) imaging, MRI, PET (Positron emission tomography), SPECT (Single Photon Emission Computed Tomography) or other techniques. The patient is placed on a table top also referred to as a couch top. Couch tops developed for Radiation Therapy are generally of a different configuration than those made for diagnostic imaging.

Radiosurgery is an effective tool for the treatment of abnormal lesions, such as malignant cancers. Stereotactic radio surgery ("SRS") combines the principles of stereotaxy (3-D target localization) with multiple cross-fired beams from a high-energy radiation source to precisely irradiate an abnormal lesion within a patient. This technique allows maximally aggressive dosing of the treatment target, while normal surrounding tissue receives lower, non-injurious doses of radiation.

Several SRS systems are available; including cobalt-sourced systems (also known as GAMMA KNIFE®, Elekta Instruments AB, Sweden), a Swedish and linear accelerator ("LINAC") based devices, such as modified linear accelerators and frameless SRS (e.g., CYBERKNIFE®, Accuray, Sunnyvale, Calif.).

GAMMA KNIFE® employs radioactive cobaltbased gamma ray, whereas LINAC-based systems use X-ray beams generated from a linear accelerator. As a result, the LINAC-based devices do not require or generate any radioactive material. One disadvantage associated with GAMMA KNIFE or conventional LINAC radiosurgery is that a metal head frame is required to be attached to the skull of a patient undergoing brain surgery, and is used to precisely target the radiation beam.

The most advanced LINAC-based system is frameless SRS, which incorporates a miniature linear accelerator mounted on a robotic arm to deliver concentrated beams of radiation to the treatment target from multiple positions and angles. Frameless SRS also employs a realtime x-ray-based image-guidance system to establish the position of the treatment target during treatment, and then dynamically brings the radiation beam into alignment with the observed position of the treatment target. Thus, frameless SRS is able to compensate for patient movement without the need for the invasive and uncomfortable head frame to ensure highly accurate delivery of radiation during treatment. As result, the patient's treatment target receives a cumulative dose of radiation high enough to control or kill the target cells while minimizing radiation exposure to surrounding healthy tissue. With sub-millimeter accuracy, frameless SRS can be used to treat tumors, cancers, vascular abnormalities and functional brain disorders. Frameless SRS can achieve surgical-like outcomes without surgery or incisions. Through the combination of a flexible robotic arm, LINAC, and image guidance technology, frameless SRS is able to reach areas of the body that are unreachable by other conventional radiosurgery systems, including the prostate.

When areas of the body outside the brain are targeted by radiosurgery, the technique is sometimes alternatively referred to as SBRT-stereotactic body radiotherapy. The terms "SRS" and "SBRT" have been used interchangeably by different practitioners to describe the same medical procedure.

Radiosurgery differs from conventional radiotherapy in several ways. The efficacy of radiotherapy depends primarily on the greater sensitivity of tumor cells to radiation in comparison to normal tissue. With all forms of standard radiotherapy, the spatial accuracy with which the treatment is focused on the tumor is less critical compared with radiosurgery; because normal tissues are protected by administering the radiation dose over multiple sessions (fractions) that take place daily for a period of a few weeks. This form of radiation is more effectively repaired by normal tissues, whereas radiosurgery is far more likely to ablate all normal tissues in the high dose zone. As such, radiosurgery, by its very definition, requires much greater targeting accuracy. With SRS, normal tissues are protected by both selective targeting of only the abnormal lesion, and by using cross-firing techniques to minimize the exposure of the adjacent anatomy. Since highly destructive doses of radiation are used, any normal structures (such as nerves or sensitive areas of the brain) within the targeted volume are subject to damage as well. Typically, SRS is administered in one to five daily fractions over consecutive days. Nearly all SRS is given on an outpatient basis without the need for anesthesia. Treatment is usually well tolerated, and only very rarely interferes with a patient's quality of life. Accordingly, SRS has been used to treat benign and malignant tumors, vascular malformations, and other disorders with minimal invasiveness.

Radiosurgical treatments generally involve several phases. First, a three-dimensional map of the anatomical structures in the treatment area is constructed using an imaging technique, such as PET scanning, SPECT, perfusion imaging, tumor hypoxia mapping, angiogenesis mapping, blood flow mapping, cell death mapping, CT, or MRI. Generally, imaging methods, such as CT or nuclear spin tomography, are used to determine the outer contours of the region to be irradiated, such as an outer contour in most cases being marked on the tomographic images obtained. Next, a treatment plan is developed to deliver a dose distribution according to the three-dimensional map. Finally, a patient is treated according to the treatment plan with an appropriate radiosurgical technique. When performing fractionated radiotherapy, accuracy in applying the radiation is very important. Some tumors or other conditions require that the radiation be concentrated in relatively small volumes. Misalignment of the radiation beam may cause an insufficient amount of radiation to be applied to the tumor or other target. Further, such misalignment may increase the likelihood and/or degree of damage to healthy tissue adjacent the tumor or other target.

Fractionated radiotherapy may be imprecise if the tumor or other target cannot be localized with a sufficient degree of accuracy. However, this need for proper localization is the same need which one has when performing single dose radiotherapy and this need is addressed by the present inventors' two first listed below patents. The additional factor in fractionated radiotherapy is the need to easily and accurately repeat a position of the patient. If the position of the patient was accurate relative to the first treatment, and relative to the imaged data set used to design the treatment, the repositioning should normally cause the patient to assume the exact same position (relative to the treatment mechanism) for the second and subsequent treatments. However, if the second or other subsequent treatment is performed with the patient only slightly moved from the first treatment position, this will introduce inaccuracies. The repeat fixation techniques discussed above have the indicated disadvantages.

More generally, the need for repeat fixation of a patient or portion of a patient exists outside of radiotherapy. In the general case, one wishes to perform a first medical procedure on a patient with a precise localization of portions of the patient, and, at some later time, perform a second medical procedure on the patient with a precise localization of portions of the patient. One can repeat laborious and time-consuming localization steps for the second medical procedure, but this increases medical costs and complexity. As used herein, a medical procedure is a procedure for diagnostic and/or remedial purposes.

In some situations, a single medical treatment without a need for repeat fixation is the desired course of treatment. However, a high degree of accuracy in positioning may still be required. The mechanical arrangements and the associated techniques of the present inventors' last mentioned above two above patents can provide a high degree of accuracy in positioning of the patient relative to the medical apparatus.

In the radiotherapy procedure, once the reference frame has been removed from the patient the relationship between intracranial target points and the reference system is lost. Because the above procedure would require the reference frame to remain fixed to the patient's skull through the entire course of treatment, which may las several weeks, this approach is considered inappropriate for fractionated therapy. Alternatively, each fractional treatment would require a laborious and time-consuming procedure to re-determine patient position for second and subsequent treatments.

Another setting would be where the patient undergoes a medical imaging procedure to be followed by stereotactic or optic guided surgery. In this setting the patient is scanned prior, sometimes a day or two before, the surgical procedure. The registration of the patient's scan data set to their position on the operative setting is carried out through the use of surface fiducials. This usually entails the shaving of the patient head.

There exist several different techniques for non-invasive repeat fixation. These methods can be broken down into three basic categories. These are bite plate systems, contour realignment systems and mask systems. All of these systems have design flaws which can lead to unacceptable, and undetectable, positional errors.

Diagnostic exams may include a set of fiducial markers whoch allow all points within the image to be localized relative to the stereotactic reference frame. All of these fiducial system attach to the stereotaxic frame in a precision and reproducible manner.

The document US5027818 describes for example techniques for providing stereotactic radiosurgery with a high degree of precicion, by allowing the patient to be precisely positioned relative to radiation beams of stereotactic radiosurgery to within 0.2 mm plus or minus 0.1 mm. These techniques work for single fraction therapy, but are not optimal for clinical settings where fractionating the total dose.

In document US 5,954,647 various locators using bite plates, head rings, and/or masks are described. The bite plates are used for repeat fixation medical procedures (i.e., where procedure is performed with locator on patient, locator and any other associated parts are then removed from the patient, and, at a later time, locator is placed back on patient).

Document US20080031414 describes a patient couch top or device for quickly and accurately positioning a patient during simulation and treatment by placing a series of small fiducial markers in discrete locations on the couch top or device. The use of the fiducial markers allows for the correction for misalignment and deformation of patient positioning equipment which occurs due in part to a patient's size and weight. This document also describes a method for positioning a patient and correcting for deformation of the couch top or device.

However, a high degree of accuracy in positioning may still be required. Accordingly, a need exists to develop markers for deployment and implantation in soft tissues, such as bronchi or bronchioli.

### Description of the Invention

The present invention provides a medical device for an intracorporeal location, comprising a deformable armature, at least partially made of a shape memory material and having an Ea end and an Eb end, said Ea end being larger than said Eb end.

More particularly, the present invention relates to a medical device for an intracorporeal location characterized in that:
- it comprises a deformable armature, at least partially made of a shape memory material and having at least one Ea end and at least one Eb end, said Ea end being larger than said Eb end,
- it has a three-dimensional shape which is essentially triangular, trapezoidal or diamond-shaped, the sides of which extending between said Eb end and the corners of said Ea end,
- said deformable armature includes a middle rip extending from said Ea end to said Eb end and multiple cross elements extending between the middle rip and said sides, wherein said sides are formed by sides braces,
- it comprises at least one x-ray visible marker fixed at the deformable armature selected from the group consisting of gold, silver, platinum, tantalum, tungsten, niobium, palladium, iridium,
- it comprises a guide suited for the intracorporeal introduction of said medical device, and an extraction holder of the medical device.

"Memory shape material" refers, according to the invention, to any materials that can return to some previously defined shape, volume or size when subjected to the appropriate thermal procedure. Advantageously, the memory shape material may be deformed at ambient temperature, and may recover its original shape when exposed to intra-body temperature. Advantageously, the medical device may be deformed at ambient temperature to adapt its shape to penetrate and circulate into the body and to attain the target area, for example a tumor or an organ. Adavntageously, the memory shape material is auto-expandable and allows the expansion of the volume of the medical device once returned to the previously defined shape, size or volume. For example, the deformed shape may be adapted to a circulation of the medical device into the blood vessels or into the lumen of a body cavity, or into a guide suited for the intracorporeal introduction, for example a catheter. For example, the deformed shape may be for example a diamond-shape, an oval-shape, a trapeze or a triangle. In this case, the deformed shape may have a length comprised between 2 and 50 mm, for example between 5 and 40 mm, or for example between 10 and 30 mm, and may have a circular, conical or oval base having a surface comprised between 0.1 cm2 and 30 cm2, for example between 0.5 and 20 cm2, or for example between 1 and 10 cm2. The medical device returns to its original shape with an Ea end and an Eb end, the Ea end being larger than said Eb end, when it is implanted into the body at the target site. Advantageously, stent deployment immediately makes keeping up grace to its structural form.

The target area may be any organ, eventually comprising a tumor, or a zone of the body comprising an inflammation or an infection, or any condition in which the medical device of the invention could be used to diagnose or treat the condition. The target organ may be chosen among the lung, the liver, kidney, bladder, adrenal glands, pancreas, Lymph nodes, brain, prostate, genitals, thyroid, mesenchymal or cutaneous tumors.

The deformable armature may define a surface between its Ea end and Eb ends. Advantageouly, the surface is larger when the deformable armature is expanded in its original shape than when the armature is in its deformed shape.

Advantageously, memory shape material is made of an alloy, for example nickel-titanium alloy, iron-platinum alloy, copper alloy systems, gold-cadmium alloy, nickel-aluminum alloy.

Eventually, the medical device may comprise a predetermined breaking point. The predetermined breaking point may be on the armature of the medical device. Advantageously, the predetermined breaking point make the implantation into the body or the withdrawal of the medical device outside of the body easier.

The medical device may comprise a mean of fastening and or/opening providing the predetermined breaking point. The mean of fastening and/or opening may be any system allowing sides of the armature of the medical device to stretch out or to come closer. For example, the mean of fastening and/or opening may be made of a metal ball joint with siliconne precut. Advantageously, the fastening and or/opening make the implantation into the body or the withdrawal of the medical device outside of the body easier. At least one x-ray visible marker is fixed at the deformable armature. The x-ray visible marker may be selected from the group including, but not limited to, gold, silver, platinum, tantalum, tungsten, niobium, palladium, iridium. For example, one, or two, or three, or at least four x-ray visible marker(s) may be fixed at the deformable armature. For example, the x-ray visible marker may be any radiopaque marker.

Advantageously, the armature may include branches for the fixing of the x-ray visible marker. The armature may comprise 1, 2, 3, 4 or more branches. Advantageously, at least one x-ray visible marker may be fixed on each of the branches. Advantageously, when the armature comprise markers on several branches, for example 3 branches, this allows a 3-D tracking of the target organ, and eventually of a tumor in the target organ.

The medical device may include a deformable material forming a surface of the medical device.

"Deformable material" refers, to any material that may change its shape when the memory shape material change its shape. In other words, the deformable material may stretch out or retract depending on the shape of the memory shape material. The deformable material may cover all or part of the surface of the medical device. The deformable material may be micro-perforated. Alternatively, the deformable material may be porous. Advantageously, the microperforations or porosities comprise one or more active substance chosen among the group comprising a therapeutic substance or a diagnostic substance. Advantageously, the active substance is released at the site of a tumor or near the site of a tumor into the body. Advantageously, the diagnostic substance may be any substance that may allow to detect or identify, once the medical device is outside the body, a condition to be treated. Whatever the shape of the medical device according to the invention, the deformable material may extend membranous between spaces of the armature to form a concave sail. For example, the membranous material may be silicone.

The deformable material may comprise a fragility zone. The fragility zone may be any area of the deformable material that does not have the same robustness than the rest of the deformable material. In other words, the fragility zone may be any area on the deformable material that is more deformable than the rest of the deformable material. For example, the fragility zone may be an area on the deformable material that does not have the same density than the rest of the deformable material, for example a pre-cut zone or a pre-perforated zone. Advantageously, the fragility zone may join the predetermined breaking point, in order to make the implantation into the body or the withdrawal of the medical device outside of the body easier. The fragility point may be a pre-cut zone at the predetermined breaking point.

The medical device may have a concave curvature. In other words, the armature of the medical device may have a concave curvature when it is stretched out. In this case, the deformable surface also has a concave curvature. Advantageously, the concave curvature allows the medical device to be adapted to the shape of organs of tumors, for example to follow all or part of the tumor or organ and be spatialy visible in a lateral view.

The medical device has an essentially triangular, diamond, oval, or a trapezoidal shape. This shape corresponds to the shape when no force is applied to the armature. In other words, this shape corresponds to the shape when the armature is not deformed. Advantageously, the shape of the medical device allows that the medical device be around the target organ. Advantageously, the target organ is inside the medical device, especially inside the armature of the medical device. Advantageously, the shape of the medical device avoid the displacement, also called migration, especially forward and/or backward, of the medical device, once the medical device is positioned properly at the target organ, especially around the targeted organ. Advantageously, the medical device may have an essentially diamond, oval, triangular or trapezoidal shape, with the Eb end forming the top side of said essentially triangular, diamond, oval or trapezoidal shape, wherein the Ea end forms the base of said essentially triangular, diamond, oval or trapezoidal shape located opposite the Eb end and having sides extending between the Eb end and the corners of the Ea end, wherein the essentially triangular, diamond, oval or trapezoidal area spanned between the sides and the base is concavely curved.

In a preferred embodiement of the invention, the shape of the medical device is the diamond shape, in other words a lozenge or rhombus shape. This shape particularly allows that the medical device be around the target organ. Advantageously, this shape of the medical device avoid the displacement, especially forward and/or backward, of the medical device, once the medical device is positioned properly at the target organ, especially around the targeted organ.

When the medical device have an essentially triangular or trapezoidal shape, the sides of the essentially triangular or trapezoidal shape extend between said Eb end and the corners of said Ea end.

Advantageously, the sides may be curved outwards. The essentially triangular, diamond, oval or trapezoidal shaped armature includes a middle rip extending from said Ea end to said Eb end and multiple cross elements extending between the middle rip and said sides, wherein the sides are formed by side braces. Advantageously, the Ea end may be formed by at least two base struts connected at the corners of the Ea and run with a spacing to each other in a middle section of the Ea end to form in combination wih the Eb end a 3-dimensional structure of the armature.

Medical device according to any one of the preceding claims, having a height in the range of 2 and 50 mm. The Ea end of the medical device in its original shape may have a length comprised between 2 and 50 mm.

The sides extending between the Eb end and the corners of the Ea end may have a length comprised between 2 and 50 mm, for example between 5 and 40 mm, or for example between 10 and 30 mm, when the medical device is in its original shape.

The medical device may comprise a system of radio guidance or a transponder. Advantageously, radio guidance or transponder helps better deliver radiation therapy to cancerous tumors, in particular since tumors can move between treatments due to differences in organ filling or movements while breathing. Transponder or radio guidance, as image-guided radiation therapy (IGRT), may involve conformal radiation treatment guided by specialized imaging tests, such as CT scans, ultrasound or X-rays. Transponder or radio-guidance as IGRT may be used during the process of two and three-dimensional imaging, during a course of radiation treatment, to direct radiation therapy utilizing the imaging coordinates of the actual radiation treatment plan. Advantageously, the patient is localized in the treatment room in the same position as planned from the reference imaging dataset. An example of Three-dimensional (3D) IGRT would include localization of a cone-beam computed tomography (CBCT) dataset with the planning computed tomography (CT) dataset from planning. Similarly Two-dimensional (2D) IGRT would include matching planar kilovoltage (kV) radiographs fluoroscopy or megavoltage (MV) images with digital reconstructed radiographs (DRRs) from the planning CT. Advantageously, combination of the radio-guidance and the transponder with x-ray visible marker allows to know exactly where the prostate is at any given time and increase the success of treatment while minimizing exposure to nearby tissues.

The medical device may comprise an active system intended for diagnosis and/or treatment of a disease. The disease may be a cancer, an inflammation, or an infection. The active system may be chosen among chimiotherapy, a reactive group specific to a substrate, a electromagnetic system for applying electromagnetic radiation or pulsed electromagnetic fields, and a biochemical or mechanical sensor, without this list being limitative. For example, the reactive group specific to a substrate may be an antibody or an antibody fragment selected in the group constituted of Fab, Fab', F(ab')₂, and Fv fragments. The active system may be fixed on the surface defined by the armature.

Another object of the disclosure is a device for the introduction, by an endoscopic or percutaneous way, of the medical device as defined above, comprising a guide suited for the intracorporeal introduction, and an extraction holder of the medical device. Advantageously, the medical device circulate inside the device for the introduction towards the target site in the body in its deformed shape, and may recover its original shape once its has attained the target site in the body.The guide may be a catheter for a percutaneous, endoscopic or laparoscopic introduction.

Another object of the disclosure is a method for accurately targeting a target area during radiation treatment through image guidance comprising determining the location of a medical device as defined above in real time using at least one method of targeting selected from the group consisting of lasers, visual, infrared, MRI/MRS, RF and radiation; and modifying the radiation treatment beam path to adaptively compensate for a change in position of the target area.

For example, a medical method of treatment of a patient in need thereof may comprise the steps, not necessarily in order, of :
- positioning a patient for a first medical procedure;
- positioning the medical device of the invention a first time to get precise positioning information relative to at least part of the patient;
- registering the exact position of the medical device relative to a portion of the patient in a registry;
- performing a first medical procedure on the patient;
- after the first medical procedure, removing the medical device from the patient;
- at a later time after the removing of the medical device, reattaching the medical device to the patient, the medical device again being in registry with the portion of the patient and having an identical relative to the portion of the patient as when the medical device was previously deployed;
- after the re-deployment step, using the medical device a second time to get precise positioning information relative to the at least part of the patient; and
- after the re-deployment step, performing a second medical procedure on the patient.

Another object of the disclosure is a method for diagnosing or treating a condition of a patient in need thereof, said method comprising the location into the body of said patient of the medical device of the invention.

The method for diagnosing a disease in a patient may comprise the location of the medical device of the invention into the body of the patient, at a target area suspected to be affected by a disease. The invention is defined in the claims, other embodiments being merely exemplary. The invention is further illustrated by the following examples with regard to the annexed drawings not be construed as limiting.

### Description of the Figures

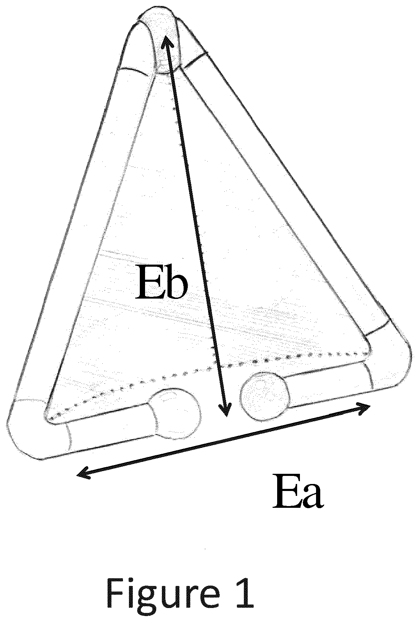

- **Figure 1****:** shows a front view of the sail stent, with the Ea end and the Eb end.

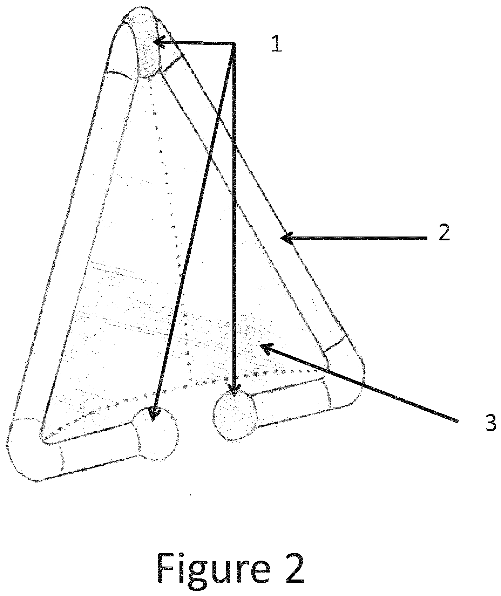

- **Figure 2****:** shows a front view of the sail stent, with X-ray visible markers (1), an memory shape armature (2) and a silicone membranous material (3) for the elution of active products or communicating systems. The membranous material is pre-cut for its extraction ("Real Eaze System").

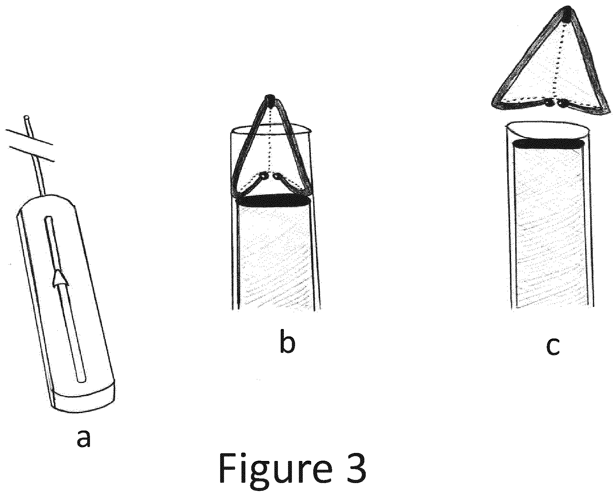

- **Figure 3****:** shows the delivery system of the stent. (a): catheter with handle for dropping of the stent. (b) front view of the catheter containing the stent and of the sail stent. (c): front view of the catheter after deployment of the stent.

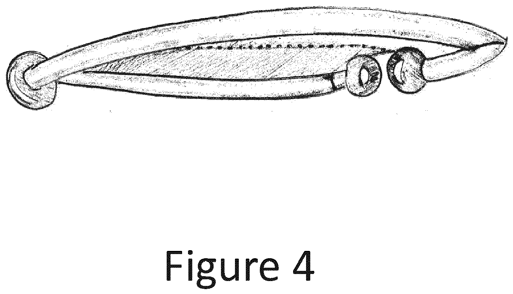

- **Figure 4**: shows a view from the side of the sail stent.

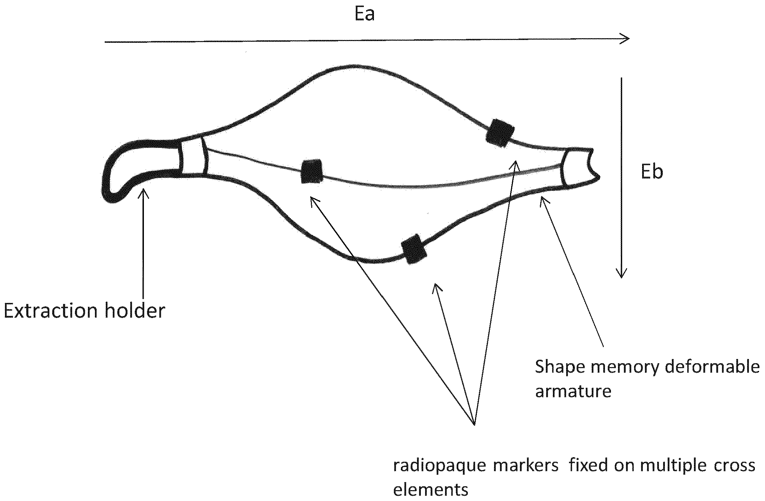

- **Figure 5**: shows a view from the side of an ovaly stent of diamond shape, comprising a shape memory deformable armature, radiopage markers fixed on multiple cross elements and the extraction holder.

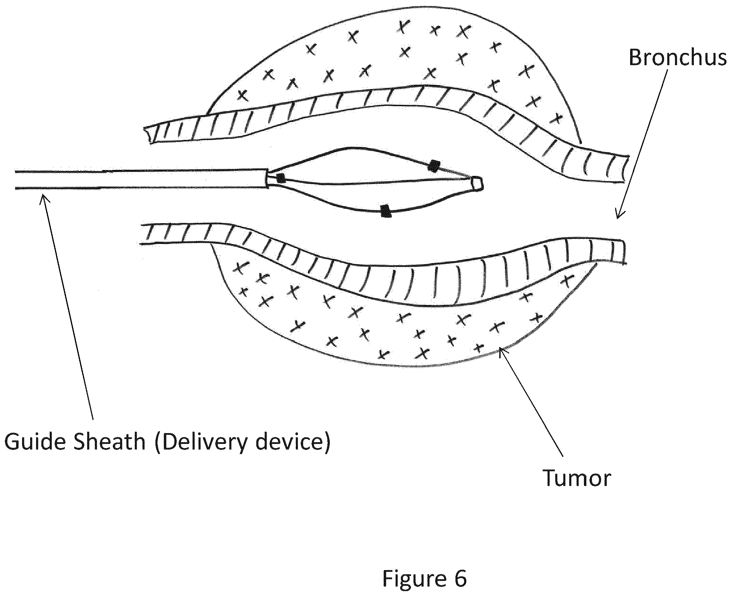

- **Figure 6****:** shows the ovaly stent launched in a tumor, comprising a guide sheath outside a delivery device.

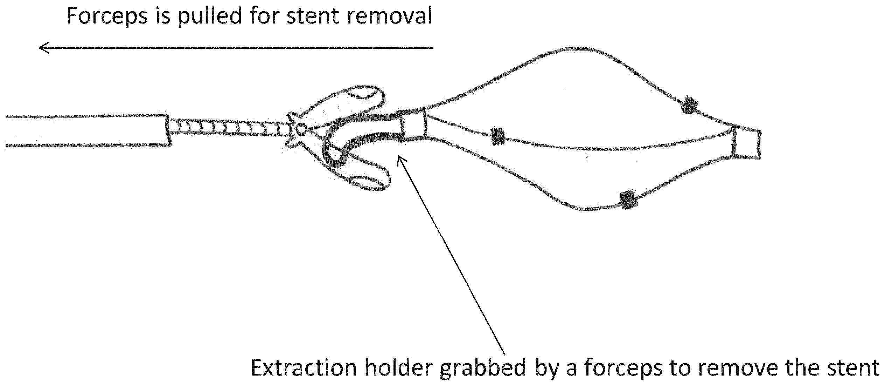

- **Figure 7****:** shows an ovaly stent removal, with an extraction holder grabbed by a forceps to remove the stent, and forceps that is pulled for stent removal.

### Examples

### Example 1: example of a clinical case

Patient of 58 years, smoking, with a chest opacity of the right upper lobe, is admitted.

PET is positive in the ipsilateral hilar and mediastinal. The rest of the staging does not find any extra thoracic lesion.

The diagnosis of squamous cell carcinoma is confirmed by flexible bronchoscopy using a technique of distal guide ultrasound mini probe. Mediastinal staging is made by an Endobronchial Ultrasound. Therapeutic decision making in multidisciplinary staff is a radio concomitant chemotherapy for stage IIIA disease classified.

Before the medical treatment, system sail stent is positioned within the tumor endoscopically.

The procedure is performed under general anesthesia. The bronchoscope goes through natural means. The same way as for the diagnosis, the lesion is found through ultrasound guidance system. The catheter is left in place within the tumor. The delivery system is inserted into the guide catheter. The sail stent is deployed within the tumor. Confirmation of correct positioning of the stent method is verified by fluoroscopy or ultrasound. The bronchoscope is removed. Procedure time did not exceed 20 minutes. The patient is awake and can enjoy a guided radiotherapy more precisely by the Sail Stent. After the treatment séquence, the stent is left in place or removed endoscopically.

The outlook is based on the possibility of increasing the intensity of radiation complications limiting the healthy tissue. In addition, the use of the stent chemotoxic by elution improve the performance of radiation.

### Example 2: Animal testing

The aim of this study is to evaluate :
(1) whether it is possible to implant the Sail fiducial markers using Sail Delivery Devices and standard endoscopic instruments (guide sheath, endoscope...),
(2) whether it is possible to reach the target locations reachable,
(3) whether there any direct complications (bleeding, pneumothorax, migration) after the implantation of the Sail Stent,
(4) if the Sail fiducial markers are visible on radiographic images, and whether it is possible to differentiate the 3 Tantalum markers,
(5) whether there is a short term migration (already after few days) of the Sail implants.

### MATERIEL AND METHODS

Pig is anesthetized and ready for the procedure (t=0). Then, an insertion of the video endoscope and the visualisation of the lung was realized. The determination of a proximal target location where it is possible to have a direct endoscopic vision was realized.

The delivery test was realized by performing the steps below:
(1) Insertion of the guide sheath in the endoscopic operating channel,
(2) Loading of the Sail fiducial markers in a Sail Delivery Device (DD),
(3) Insertion of the Sail DD within the guide sheath,
(4) Performing a Radiographic control to visualize the Sail DD tip position
(5) Pushing the pusher to release the Sail fiducial marker,
(6) Visualization simultaneously on the operating room screen how the Sail fiducial marker is released in the lung,
(7) Removing the Sail DD and let the guide sheath in position,
(8) Insertion of the Olympus biopsy Forceps FB-233D in the guide sheath,
(9) Opening of the biopsy forceps and catching the Sail fiducial marker proximal handle,
(10) Pull very carefully the biopsy forceps,
(11) Then it is decided to let the Sail fiducial marker in place or to remove it.

Other proximal tests with the other Sail DD and/or others implants are performed ; the proximal tests should not last longer than 15/20 minutes.

Then, the distal targets location are determined and delivery tests are performed.

At the end of the procedure, 10 Sail fiducial markers (4 in the right and 6 in the left lung) were implanted.

Then, a fluoroscopy is performed.

The procedure is finished, and has lasted 1 hour 30.

Depending on the success of the intervention, the pigs are not euthanized and are kept alive for few days in order to evaluate eventual complications due to the Sail fiducial markers.

After this few days a fluoroscopy is performed to compare the position of the Sail fiducial markers and evaluate the migration risks. It is not possible to perform an endoscopic control.

### RESULTS

The stent delivery was succesfull, even in the proximal or in the distal airways. We succesfully tested the device withdrawal by removing the stent in the Guide Sheath with the forceps grabing the extremity handle (Extraction holder).

The 10 stents were succesfully implanted in the pigs. The control with fluoroscopy is satisfaying. The pigs were awake and monitored (clinicaly) during one week.

At the end of the period, the animals were euthanized. Just before this step, we proceed a Fluoroscopy that confimed the absence of stent migration. There were no clinical complications during the Week of monitoring (No pneumothorax, Hemoptysis, pneumonia, fever, ofr death...).

### List of references

- US5027818.
- US 5,954,647.
- US20080031414.

## Claims

1. Medical device for an intracorporeal location, comprising:
- a deformable armature (2), at least partially made of a shape memory material and having at least : a first end (Ea), a second end (Eb), sides and corners;
said first end (Ea) forming the base of said medical device, said second end (Eb) forming the top side of the medical device, said first end (Ea) being larger than said second end (Eb),
the sides of the armature extending between said second end (Eb) and the corners of said first end (Ea);
wherein said medical device has a shape chosen among essentially triangular or trapezoidal;
- at least one x-ray visible marker fixed at the deformable armature selected from the group consisting of gold, silver, platinum, tantalum, tungsten, niobium, palladium, iridium,
- a guide suited for the intracorporeal introduction of said deformable armature;
- and an extraction holder of said deformable armature;
the medical device **characterised in that**:
- said deformable armature (2) includes a middle rip extending from said first end (Ea) to said second end (Eb) and multiple cross elements extending between the middle rip and said sides, wherein said sides are formed by side braces.

2. Medical device according to claim 1, including a deformable material forming a surface of the medical device.

3. Medical device according to anyone of claims 1 to 2, having a concave curvature.

4. Medical device according to anyone of claims 1 to 3, having an essentially triangular or trapezoidal shape, with the second end (Eb) forming the top side of said essentially triangular or trapezoidal shape, wherein the first end (Ea) forms the base of said essentially triangular or trapezoidal shape located opposite the second end (Eb) and having sides extending between the second end (Eb) and the corners of the first end (Ea), wherein the essentially triangular or trapezoidal area is spanned between the sides and the base is concavely curved.

5. Medical device according to anyone of the preceding claims, comprising a predetermined breaking point.

6. Medical device according to claim 5, comprising a mean of fastening and or/opening providing said predetermined breaking point.

7. Medical device according to claim 6, in which said mean of fastening and/or opening is constituted of a metal ball joint.

8. Medical device according to any one of claims 2 to 7, in which said deformable material is micro-perforated.

9. Medical device according to any one of claims 2 to 8, in which said deformable material comprises a fragility zone.

10. Medical device according to any one of claims 3 to 9, in which said deformable material comprises an active substance chosen among the group comprising a therapeutic substance or a diagnostic substance.

11. Medical device according to any one of the preceding claims, comprising a system of radio guidance or a transponder.

12. Medical device according to any one of the preceding claims, wherein said first end (Ea) has a length comprised between in the range of 2 to 50 mm.

## Patentansprüche

1. Medizinische Vorrichtung für eine intrakorporale Verbringung, die umfasst:
• eine verformbare Armatur (2), die zumindest zum Teil aus einem Formgedächtnismaterial besteht und die zumindest ein Ende (Ea), ein zweites Ende (Eb), Ecken und Seiten aufweist,
wobei das besagte erste Ende (Ea) die Basis der medizinischen Vorrichtung bildet,
wobei das besagte zweite ende (Eb) die oberseite der medizinischen Vorrichtung bildet,
wobei das besagte erste Ende (Ea) größer als das besagte zweite Ende (Eb) ist,
wobei die Seiten der Armatur (2) sich zwischen dem besagten zweiten Ende (Eb) und den Ecken des besagten ersten Endes erstrecken,
wobei die besagte medizinische Vorrichtung eine dreidimensionale Form hat, die unter im wesentlichen dreieckigen oder trapezförmigen Formen gewählt ist,
• zumindest einen röntgensichtbaren Marker, der an der verformbaren Armatur befestigt ist und aus der Gruppe bestehend aus Gold, Silber, Platin, Tantal, Wolfram, Niob, Palladium, Iridium ausgewählt wurde,
• eine für die intrakorporale Einführung der verformbaren armatur, und
• einen Extraktionshalter der verformbaren Armatur,
**dadurch gekennzeichnet, daß** die besagte verformbare Armatur eine mittlere Sicke enthält, die sich vom besagten ersten Ende (Ea) zum besagten zweiten Ende (Eb) erstreckt, und mehrere Querelemente, die sich zwischen der mittleren Sicke und den besagten Seiten erstrecken, wobei die besagten Seiten von Seitenklammern gebildet warden,

2. Medizinische Vorrichtung nach Anspruch 1, die ein verformbares Material enthält, das eine Fläche der medizinischen Vorrichtung bildet.

3. Medizinische Vorrichtung nach einem beliebigen der vorstehenden Ansprüche 1 bis 2, die eine konkave Krümmung aufweist.

4. Medizinische Vorrichtung nach einem beliebigen der vorstehenden Ansprüche 1 bis 3, die eine im wesentlichen dreieckige oder trapezförmige Form aufweist, dabei das Zweite Ende (Eb) die Oberseite der besagten im wesentlichen dreieckigen oder trapezförmigen Form bildet, wobei das erste Ende (Ea) die Basis der besagten im wesentlichen dreieckigen oder trapezförmigen Form bildet, die sich gegenüber dem ersten Ende (Eb) befindet und Seiten hat, die sich zwischen dem zweiten Ende (Eb)und den Ecken des ersten Enden (Ea) erstrecken, wobei der sich zwischen den Seiten und der Basis überspannende im wesentlichen dreieckige oder trapezförmigeBereich konkav gekrümmt ist.

5. Medizinische Vorrichtung nach einem beliebigen der vorstehenden Ansprüche, die eine Sollbruchstelle umfasst.

6. Medizinische Vorrichtung nach Anspruch 5, die ein Mittel zur Befestigung und/oder Öffnung umfasst, das die besagte Sollbruchstelle bereitstellt.

7. Medizinische Vorrichtung nach Anspruch 6, in der das besagte Mittel zur Befestigung und/oder Öffnung aus einem Metallkugelgelenk besteht.

8. Medizinische Vorrichtung nach einem beliebigen der vorstehenden Ansprüche 2 bis 7, in der das besagte verformbare Material mikroperforiert ist.

9. Medizinische Vorrichtung nach einem beliebigen der vorstehenden Ansprüche 2 bis 8, in der das besagte verformbare Material einen Zerbrechlichkeitsbereich umfasst.

10. Medizinische Vorrichtung nach einem beliebigen der vorstehenden Ansprüche 3 bis 9, in der das besagte verformbare Material eine Aktivsubstanz umfasst, die aus einer eine therapeutische Substanz oder eine Diagnosesubstanz umfassenden Gruppe ausgewählt wurde.

11. Medizinische Vorrichtung nach einem beliebigen der vorstehenden Ansprüche, die ein Funkleitsystem oder einen Transponder umfasst.

12. Medizinische Vorrichtung nach einem beliebigen der vorstehenden Ansprüche, wobei das besagt erste Ende (Ea) eine Höhe im Bereich von 2 bis 50 mm aufweist.

## Revendications

1. Dispositif médical pour un placement intracorporel, comprenant :
- une armature déformable (2), au moins en partie faite d'un matériau à mémoire de forme et ayant au moins :
une première extrémité (Ea), et une seconde extrémité (Eb), des côtés et des coins ;
ladite première extrémité (Ea) formant la base dudit dispositif médical, ladite seconde extrémité (Eb) formant la partie supérieure du dispositif médical, ladite première extrémité (Ea) étant plus grande que ladite seconde extrémité (Eb),
les côtés de l'armature s'étendent entre ladite seconde extrémité (Eb) et les coins de ladite première extrémité (Ea) ;
dans lequel ledit dispositif médical a une forme choisie parmi une forme essentiellement triangulaire ou trapézoïdale ;
- au moins un marqueur visible aux rayons X fixé sur l'armature déformable choisi dans le groupe consistant en l'or, l'argent, le platine, le tantale, le tungstène, le niobium, le palladium, l'iridium,
- un guide approprié pour l'introduction intracorporelle de ladite armature déformable ;
- et un support d'extraction de ladite armature déformable ;
le dispositif médical étant **caractérisé en ce que** :
- ladite armature déformable (2) comporte une entaille médiane s'étendant depuis ladite première extrémité (Ea) à ladite seconde extrémité (Eb) et de multiples éléments transversaux s'étendant entre l'entaille médiane et lesdits côtés, dans lequel lesdits côtés sont formés par des entretoises latérales.

2. Dispositif médical selon la revendication 1, comportant un matériau déformable formant une surface du dispositif médical.

3. Dispositif médical selon l'une quelconque des revendications 1 à 2, ayant une courbure concave.

4. Dispositif médical selon l'une quelconque des revendications 1 à 3, ayant une forme essentiellement triangulaire ou trapézoïdale, la seconde extrémité (Eb) formant la partie supérieure de ladite forme essentiellement triangulaire ou trapézoïdale, dans lequel la première extrémité (Ea) forme la base de ladite forme essentiellement triangulaire ou trapézoïdale située à l'opposé de la seconde extrémité (Eb) et ayant des côtés s'étendant entre la seconde extrémité (Eb) et les coins de la première extrémité (Ea), dans lequel la zone essentiellement triangulaire ou trapézoïdale étendue entre les côtés et la base est courbée sur le plan concave.

5. Dispositif médical selon l'une quelconque des revendications précédentes, comprenant un point de rupture prédéterminé.

6. Dispositif médical selon la revendication 5, comprenant un moyen de fermeture et/ou d'ouverture permettant ledit point de rupture prédéterminé.

7. Dispositif médical selon la revendication 6, dans lequel ledit moyen de fermeture et/ou d'ouverture est constitué d'une articulation métallique sphérique.

8. Dispositif médical selon l'une quelconque des revendications 2 à 7, dans lequel ledit matériau déformable est micro-perforé.

9. Dispositif médical selon l'une quelconque des revendications 2 à 8, dans lequel ledit matériau déformable comprend une zone de fragilité.

10. Dispositif médical selon l'une quelconque des revendications 3 à 9, dans lequel ledit matériau déformable comprend une substance active choisie dans le groupe comprenant une substance thérapeutique ou une substance diagnostique.

11. Dispositif médical selon l'une quelconque des revendications précédentes, comprenant un système de radioguidage ou un transpondeur.

12. Dispositif médical selon l'une quelconque des revendications précédentes, dans lequel ladite première extrémité (Ea) a une longueur comprise dans la plage de valeurs de 2 à 50 mm.
